# FASCICULE DE BREVET EUROPEEN

(11) **EP 2 583 242 B1**
(45) Date de publication et mention de la délivrance du brevet: **04.09.2019**
(21) Numéro de dépôt: 11735510.7
(22) Date de dépôt: 16.06.2011
(51) Int. Cl.: G06K 9/00, G06K 9/46, A61B 34/10, A61B 34/20, G06T 7/12

(54) **PROCEDE D'IDENTIFICATION AUTOMATIQUE DES CONTOURS D'UN OS PREDEFINI, PROCEDES DERIVES ET PRODUITS PROGRAMME D'ORDINATEUR CORRESPONDANTS**
VERFAHRE DER AUTOMATISCHEN IDENTIFIZIERUNG DER KONTUREN EINES VORDEFINIERTEN KNOCHENS, ABGELEITETE VERFAHREN UND ENTSPRECHENDE COMPUTERPROGRAMMPRODUKTE
METHOD OF AUTOMATIC IDENTIFICATION OF THE CONTOURS OF A PREDEFINED BONE, DERIVED METHODS AND CORRESPONDING COMPUTER PROGRAM PRODUCTS

(30) Priorité: 16.06.2010 US 355377 P; 16.06.2010 FR 1054785
(43) Date de publication de la demande: 24.04.2013
(73) Titulaire: IMASCAP, 29280 Plouzané (FR)
(72) Inventeur: CHAOUI, Jean, 29280 Locmaria Plouzane (FR); LAVALLEE, Stéphane, 38410 Saint Martin d'Uriage (FR)
(74) Mandataire: Lavoix
(86) Numéro de dépôt international: PCT/FR2011/051377
(87) Numéro de publication internationale: WO 2011/157961

(56) Documents cités:
- WO-A2-2008/014082
- CHIEN-CHENG LEE ET AL: "Identifying multiple abdominal organs from CT image series using a multimodule contextual neural network and spatial fuzzy rules", IEEE TRANSACTIONS ON INFORMATION TECHNOLOGY IN BIOMEDICINE, IEEE SERVICE CENTER, LOS ALAMITOS, CA, US, vol. 7, no. 3, 1 septembre 2003 (2003-09-01), pages 208-217, XP011100536, ISSN: 1089-7771, DOI: DOI:10.1109/TITB.2003.813795
- CHIEN-CHENG LEE ET AL: "Recognizing abdominal organs in CT images using contextual neural network and fuzzy rules", ENGINEERING IN MEDICINE AND BIOLOGY SOCIETY, 2000. PROCEEDINGS OF THE 22ND ANNUAL INTERNATIONAL CONFERENCE OF THE IEEE 23-28 JULY 2000, PISCATAWAY, NJ, USA,IEEE, vol. 3, 23 juillet 2000 (2000-07-23), pages 1745-1748, XP010530837, ISBN: 978-0-7803-6465-3
- KOBASHI M ET AL: "Knowledge-based organ identification from ct images", PATTERN RECOGNITION, ELSEVIER, GB, vol. 28, no. 4, 1 avril 1995 (1995-04-01), pages 475-491, XP004013165, ISSN: 0031-3203, DOI: DOI:10.1016/0031-3203(94)00124-5
- Geoff Dougherty: "Digital Image Processing for Medical Applications", 11 mai 2009 (2009-05-11), Cambridge University Press, XP002615721, ISBN: 9780521860857 pages 423-423,
- TAMEZ-PENA J G ET AL: "The integration of automatic segmentation and motion tracking for 4D reconstruction and visualization of musculoskeletal structures", BIOMEDICAL IMAGE ANALYSIS, 1998. PROCEEDINGS. WORKSHOP ON SANTA BARBARA, CA, USA 26-27 JUNE 1998, LOS ALAMITOS, CA, USA,IEEE COMPUT. SOC, US, 26 juin 1998 (1998-06-26), pages 154-163, XP010291418, DOI: DOI:10.1109/BIA.1998.692437 ISBN: 978-0-8186-8460-9
- NHAT TAN NGUYEN ET AL: "A new segmentation method for MRI images of the shoulder joint", COMPUTER AND ROBOT VISION, 2007. CRV '07. FOURTH CANADIAN CONFERE NCE ON, IEEE, PI, 1 mai 2007 (2007-05-01), pages 329-338, XP031175821, ISBN: 978-0-7695-2786-4

## Description

### 1. Domaine de l'invention

Le domaine de l'invention est celui de l'imagerie médicale.

Plus précisément, l'invention concerne un procédé d'identification automatique des contours d'au moins une portion d'un os prédéfini, un procédé d'identification de la surface d'une zone singulière d'une portion d'os et un produit programme d'ordinateur pour la mise en oeuvre de ces procédés.

L'invention trouve une application dans le domaine de la chirurgie assistée par ordinateur, consacrée par exemple à l'appareillage ou à l'arthroplastie de l'épaule. Elle concerne également un procédé de guidage d'un outil chirurgical, un procédé de simulation de la pose d'un composant d'une prothèse et un produit programme d'ordinateur pour la mise en oeuvre de ces procédés.

### 2. Etat de la technique

Pendant longtemps, les praticiens n'ont disposé que des images en deux dimensions fournies par un appareil de radiologie pour établir un diagnostic ou préparer une opération chirurgicale portant sur une région osseuse.

Depuis quelques années, les praticiens disposent de techniques de modélisation tridimensionnelle des contours des os à partir d'images obtenues par des techniques d'imagerie médicale classiques, telles que la tomodensitométrie, encore appelée scanner, ou l'IRM (acronyme d'imagerie par résonance magnétique). Toutes ces techniques connues nécessitent cependant pour être mise en oeuvre de solliciter le praticien dans des proportions plus ou moins importantes, notamment pour identifier à quels os correspondent ces contours sur chacune des images. Ceci conduit assez souvent à une appréciation approximative de la forme et de la position relative des os, qui peut entraîner des imprécisions ou des erreurs sur un diagnostic, ou un échec d'une opération chirurgicale orthopédique visant par exemple à implanter un composant d'une prothèse sur un os.

On constate que le taux d'échec est nettement plus important lorsque l'opération concerne une région articulaire masquée par des tissus et/ou dont l'accès est limité. C'est notamment le cas de l'articulation de l'épaule dont la chirurgie est rendue encore plus délicate par l'environnement ligamentaire de l'épaule.

Il existe donc une demande pour des techniques permettant d'assister ou de simuler le geste du chirurgien, d'autant que le nombre d'opérations de chirurgie osseuse ou orthopédique devrait croître de façon significative dans les pays industrialisés dans les prochaines années.

On a certes proposé des techniques informatiques d'assistance au geste opératoire pour l'opération du rachis et pour des opérations la neurochirurgie, s'appuyant sur des images radiologiques pré-opératoires.

Un inconvénient de ces techniques connues, dites de navigation chirurgicale, est qu'elles ne fournissent pas des résultats satisfaisants lorsqu'elles sont appliquées pour d'autres types opérations, et notamment pour des opérations de chirurgie orthopédique visant à poser un implant prothétique.

Un autre inconvénient de ces techniques de navigation chirurgicale connues est qu'il est nécessaire soit de faire intervenir un ingénieur support lors de l'opération chirurgicale, ce qui est très coûteux, soit de dispenser une formation spécifique à l'équipe chirurgicale pour qu'elle puisse assurer elle-même les tâches de l'ingénieur-support, ce qui en pratique est difficile à mettre en oeuvre.

L'article de Chien-Cheng Lee et al. intitulé « Identifying multiple abdominal organs from CT image series using a multimodule contectual neural network and spatial fuzzy rules » et publié dans IEEE Transactions on Information Technology in Biomedecine, vol. 7, n° 3, 1er septembre 2003, pages 208-217 décrit une technique d'identification d'organes dans des images, qui toutefois ne permet pas l'identification des contours d'un os.

### 3. Objectifs de l'invention

L'invention a donc notamment pour objectif de pallier les inconvénients de l'état de la technique cités ci-dessus.

Plus précisément l'invention a pour objectif de fournir une technique d'identification des contours d'une portion d'un os prédéfini à partir d'images obtenues par des techniques d'imagerie médicale connues en soi qui s'opère de façon automatique, ou en d'autres termes qui ne nécessite pas d'intervention humaine.

Un objectif de l'invention est également de fournir une telle technique qui soit simple et efficace.

Un autre objectif de l'invention est de fournir une telle technique qui soit fiable.

Encore un objectif de l'invention est de fournir une telle technique qui puisse être exploitée dans des conditions opératoires, en fournissant notamment des contours d'un os dans un délai convenable.

Un autre objectif de l'invention est de fournir une telle technique qui puisse permette de gérer de façon automatique, des occurrences de pertes de contour ou de fusion entre deux régions osseuses différentes, sans procéder à des modifications substantielles de mise en oeuvre de cette technique.

Un objectif de l'invention est également, dans au moins un mode de réalisation particulier de l'invention, de fournir une information représentative du capital osseux d'une région d'un os prédéfini.

Un autre objectif de l'invention est de faciliter et de rendre robuste le recalage des contours identifiés avec un repère opératoire tel que cela est nécessaire dans un système de navigation chirurgicale.

### 4. Exposé de l'invention

Ces objectifs, ainsi que d'autres qui apparaîtront par la suite sont atteints à l'aide d'un procédé d'identification automatique des contours d'au moins une portion d'un os prédéfini de l'épaule à partir d'une pluralité d'images représentant des coupes parallèles d'un volume de mesure comprenant ladite portion d'os, obtenues par une technique d'imagerie médicale, telle que la tomodensitométrie ou l'imagerie par résonance magnétique.

Dans le cadre de l'invention les images à partir desquelles sont extraits lesdits contours sont préférentiellement des coupes parallèles au plan transverse. Il peut également s'agir de coupes reconstruites parallèles au plan sagittal ou au plan frontal.

Selon l'invention, un tel procédé comprend :
- une étape de filtrage desdites images comprenant une étape de comparaison, pour chacune desdites images, de l'intensité d'au moins un point élémentaire de ladite image avec une intensité de référence, de façon à obtenir une image filtrée comprenant lesdits points élémentaires de l'image dont la densité correspond à celle d'un tissu osseux ;
- une étape d'obtention d'au moins une forme de contour fermé dans au moins une desdites images filtrées par application d'au moins un filtre morphologique auxdites images filtrées ;
- une étape d'association à chacune desdites formes d'une étiquette sélectionnée au sein d'une nomenclature prédéfinie, ladite nomenclature prédéfinie comprenant les termes os long, os plat, autres os et table d'opération dans le cas où ledit os prédéfini est un os de l'épaule et ladite étape d'association comprenant, pour chacune desdites formes, les étapes suivantes dans cet ordre :
   - une étape de comparaison d'un n-uplet de données adimensionnelles avec au moins un n-uplet prédéfini, n'étant supérieur ou égal à 2, lesdites données appartenant au groupe comprenant au moins l'élongation, la compacité, le rectangle englobant et les moments d'inertie normalisés de ladite forme de contour fermé, le rectangle englobant étant défini comme le rapport de la longueur sur la largeur d'un rectangle circonscrit dont les bords sont tangents aux contours de ladite forme ;
   - dans le cas où ledit ensemble de n valeurs est sensiblement semblable à l'un desdits n-uplets prédéfinis, une étape d'affectation à ladite forme d'une étiquette attachée audit n-uplet prédéfini, l'ensemble étant dit sensiblement semblable lorsque chacune des n valeurs diffère seulement de quelques pourcents par rapport aux valeurs d'un desdits n-uplets prédéfinis ; et
   - une étape d'affectation à chacune des formes restant sans étiquette à l'issue de ladite étape d'affectation à ladite forme d'une étiquette attachée audit n-uplet prédéfini, d'une étiquette attachée à un vecteur invariant normalisé prédéfini sélectionné parmi une pluralité de vecteurs invariants normalisés prédéfinis, minimisant la distance avec un vecteur invariant normalisé formé d'un nombre prédéterminé de coefficients normalisés représentatifs des coordonnées dudit contour dans l'espace de Fourier ;
   - une étape d'identification desdits contours de ladite portion d'os comprenant:
   - une étape de groupement desdites formes de sorte à former au moins un groupe de formes délimitant un volume commun isolé dans l'espace ;
   - une étape de détermination parmi lesdits groupes de forme, d'un groupe de formes cible pour lequel l'étiquette correspondant audit os est majoritairement associée aux formes du groupe ;
   - une étape de sélection des formes dudit groupe de formes cible.

Ainsi, de façon inédite, l'invention propose notamment d'associer automatiquement une étiquette à chaque contour fermé détecté sur une image d'une série de coupes parallèles, en la sélectionnant dans une nomenclature prédéfinie, de façon à restituer les contours d'un os donné. Ces étiquettes vont permettre de sélectionner un groupe de formes appartenant à la surface d'un même volume isolé de l'espace correspondant à l'os prédéfini recherché. En effet, les inventeurs ont astucieusement observé qu'il suffit de déterminer le groupe de formes au sein duquel les étiquettes correspondant à l'os recherché sont majoritaires, pour accéder aux contours de cet os. L'invention permet ainsi de reconnaître les contours d'un os particulier, quel qu'il soit, et quelle que soit le volume de mesure, de façon rapide, et fiable.

La mise en oeuvre de l'invention, qui est en outre automatique, ne nécessite donc en aucun cas l'expertise d'un praticien pour lever des indéterminations, ou une intervention de toute autre personne. Elle ne souffre donc pas d'un défaut de reproductibilité.

Selon un mode de réalisation particulier de l'invention, un tel procédé d'identification comprend en outre une étape de remplacement des étiquettes des formes dudit groupe cible ne correspondant pas audit os prédéfini par ladite étiquette correspondant audit os prédéfini.

Une correction automatique des anomalies d'étiquetage est ainsi prévue pour unifier le libellé des étiquettes associées à des formes se rattachant à un même os, de façon à pouvoir appeler simplement les formes de l'os recherché en utilisant l'étiquette qui lui correspond.

Préférentiellement, les étiquettes attachées aux vecteurs invariants normalisés prédéfinis appartiennent au groupe comprenant au moins : os long, os court, os allongé, os rayonné, os arqué, os papyracé, os pneumatique, os sésamoïde.

Les étapes de comparaison et d'affectation conditionnelle permettent ainsi généralement de détecter des côtes, une table d'examen ou des parties épiphysaires, de façon certaine.

Il convient de noter que le ou lesdits n-uplets prédéfinis sont préférentiellement extraits d'une base de données constituée, pour chaque type d'os, à partir de relevés anatomiques sur une grande population de patients, cette base étant validée par un expert médical.

Avantageusement, ladite étape d'association comprend une étape d'affectation à ladite forme d'une étiquette attachée à un vecteur invariant normalisé prédéfini sélectionné parmi une pluralité de vecteurs invariants normalisés prédéfinis, minimisant la distance avec un vecteur invariant normalisé formé d'un nombre prédéterminé de coefficients normalisés représentatifs des coordonnées dudit contour dans l'espace de Fourier.

On compare ainsi des descripteurs de Fourier de chaque forme avec les vecteurs d'un dictionnaire de forme de référence, ce qui permet de distinguer des os longs, d'os courts ou d'autres os, tels que les os allongés, les os rayonnés, les os arqués, les os papyracés, les os pneumatiques, ou les os sésamoïdes.

De façon avantageuse, ledit nombre prédéterminé de coefficients est supérieur ou égal à 8.

Le filtre morphologique permet d'éliminer le bruit sur les images et de conserver seulement les formes dont le contour est d'une taille suffisante pour correspondre au contour d'un os.

Dans un mode de réalisation particulier de l'invention, ledit os prédéfini est une omoplate ou un humérus.

L'invention concerne également un procédé d'identification automatique de la surface d'au moins une zone singulière telle qu'une saillie, une cavité ou une zone sensiblement plane, sur une portion d'un os prédéfini dont les contours ont été obtenus conformément à l'un quelconque des procédés d'identification automatique des contours d'au moins une portion d'un os prédéfini décrits ci-dessus.

Un tel procédé d'identification automatique de la surface d'au moins une zone singulière comprend :
- une étape d'obtention de la position tridimensionnelle d'au moins un point spécifique propre à ladite surface de ladite portion d'un os prédéfini à partir desdits contours ;
- une étape d'identification de ladite surface de ladite zone singulière dans une calotte sphérique centrée autour dudit point, de rayon et d'épaisseur prédéfinis et fonction de la distance dudit point à ladite zone singulière et d'une estimation de la taille de ladite zone singulière.

Ainsi, grâce à l'invention, une technique permettant d'identifier la surface d'une zone d'un os de façon automatique est proposée pour la première fois. Cette technique présente des intérêts remarquables, à la fois et de manière indépendante, pour optimiser la position d'implantation d'un composant prothétique préexistant au niveau d'une zone singulière d'un os, et pour fabriquer un composant prothétique spécifiquement adapté à cette zone singulière.

Cette technique originale repose sur l'identification de la "signature de forme" de la zone recherchée, ou en d'autres termes de la distance de cette zone avec un ou plusieurs points spécifiques de l'os, aisément détectables, tels que par exemple l'extrémité d'une pointe, ...

Cette zone singulière peut par exemple être l'acromion, la surface glénoïdale, ou le processus coracoïde, dans le cas d'une omoplate.

L'invention concerne aussi un procédé de détermination automatique des paramètres initiaux d'une matrice de passage entre un premier repère associé à au moins une portion d'un os d'un patient et un deuxième repère associé aux contours de ladite portion d'os identifiés sur une pluralité d'images obtenues par une technique d'imagerie médicale représentant des coupes d'un volume de mesure comprenant ladite portion d'os. Selon l'invention, ce procédé de détermination automatique des paramètres initiaux d'une matrice de passage comprend :
- une étape d'identification automatique de la surface d'une zone singulière sur lesdites images comprenant les étapes du procédé d'identification automatique de la surface d'au moins une zone singulière sur la surface d'un os décrit ci-dessus ;
- une étape de détermination du barycentre de ladite zone singulière et du vecteur normal au plan médian de ladite surface de ladite zone singulière orienté vers l'extérieur, ledit plan médian étant une approximation au sens des moindres carrés de la surface de la zone singulière ;
- une étape d'obtention de paramètres de recalage résultant de la coïncidence dudit barycentre et dudit vecteur normal déterminés avec le barycentre de ladite zone singulière numérisée à l'aide d'un laser, à l'aide d'une sonde échographique ou par palpage sur ladite portion d'os et un vecteur normal au plan médian de la surface de ladite zone singulière numérisée, orienté vers l'extérieur ;
- une étape de formation desdits paramètres initiaux à partir desdits paramètres de recalage.

On obtient ainsi une matrice de passage initiale robuste, qui permet de recaler finement le premier repère associé au patient et le deuxième repère associé à la modélisation des contours de l'os, en appliquant à cette matrice des algorithmes de minimisation de distances connus en soi. Tout risque de divergence vers des minima locaux inappropriés est écarté.

Cette matrice de passage initiale est par ailleurs obtenue de façon automatique, ou en d'autres termes sans intervention d'un praticien. Contrairement aux techniques connues, il n'est pas nécessaire de venir repérer visuellement trois points du deuxième repère sur les images de radiologie, ni de numériser ces mêmes trois points sur l'os du patient. Le praticien doit seulement veiller au préalable à numériser la surface de la zone singulière choisie, ce qui est rapide, et n'exige pas d'être précis.

Il convient de noter que dans le cadre de l'invention, on entend par les termes "numérisation" ou "digitalisation", toute technique permettant de collecter des informations sur la position tridimensionnelle d'un point ou d'une surface d'un objet ou d'un être réel. Il peut par exemple s'agir d'une technique de numérisation optique à l'aide d'un laser, d'une technique de numérisation à l'aide d'une sonde échographique, ou d'une technique de mesure tridimensionnelle à l'aide de palpeurs 3D.

L'invention concerne également un procédé de simulation de la pose d'un élément d'une prothèse au niveau d'une zone singulière d'une portion d'un os prédéfini comprenant :
- une étape d'identification automatique de la surface de ladite zone singulière sur des images de ladite portion d'un os prédéfini obtenues par une technique d'imagerie médicale comprenant les étapes du procédé d'identification automatique de la surface d'au moins une zone singulière sur une portion d'un os décrit ci-dessus ;
- une étape de détermination d'une première information comprenant le plan de référence de ladite surface, et/ou l'angle de version et/ou l'angle d'inclinaison associés à ladite surface ;
- une étape de détermination d'une deuxième information représentative du capital osseux associé à ladite surface, à partir de la donnée de ladite surface et de la donnée des contours de ladite portion d'os obtenus conformément à l'un quelconque des procédés d'identification automatique des contours d'au moins une portion d'un os prédéfini décrits ci-dessus ;
- une étape de restitution desdites première et deuxième informations en regard d'au moins un paramètre géométrique de l'élément de prothèse.

Le praticien dispose ainsi automatiquement grâce à ce procédé d'une information sur la géométrie de la zone d'implantation de l'élément de prothèse mais aussi d'une information sur le capital osseux de cette zone.

L'invention concerne encore un produit programme d'ordinateur téléchargeable depuis un réseau de communication et/ou enregistré sur un support lisible par ordinateur et/ou exécutable par un processeur comprenant des instructions de code de programme pour l'exécution des étapes d'un quelconque des procédés d'identification automatique des contours d'au moins une portion d'un os prédéfini décrits ci-dessus et/ou des étapes d'un procédé d'identification automatique d'au moins une zone singulière sur une portion d'un os prédéfini tel que décrit ci-dessus et/ou des étapes du procédé de détermination automatique des paramètres initiaux d'une matrice de passage présenté précédemment et/ou des étapes du procédé de simulation d'un élément d'une prothèse au niveau d'une zone singulière d'une portion d'un os prédéfini tel que présenté ci-dessus, lorsque ledit programme est exécuté par un ordinateur.

On définit également ici une méthode chirurgicale d'implantation d'un élément d'une prothèse au niveau d'une zone singulière d'une portion d'un os d'un patient, tel que par exemple un os d'une articulation, et en particulier de l'articulation de l'épaule, comprenant :
- une étape d'obtention par une technique d'imagerie médicale d'une pluralité d'images représentant des coupes parallèles d'un volume de mesure comprenant ladite portion d'os ;
- une étape d'identification automatique de la surface de ladite zone singulière comprenant les étapes du procédé selon l'invention d'identification automatique de la surface d'au moins une zone singulière sur une portion d'un os décrit ci-dessus ;
- une étape de solidarisation de moyens de repérage dans l'espace à ladite portion d'os, destinés à associer un premier repère à ladite portion d'os ;
- une étape de numérisation de ladite zone singulière ;
- une étape de recalage automatique dudit premier repère avec un deuxième repère associé aux contours de ladite portion d'os identifiés à partir de ladite pluralité d'images par la mise en oeuvre d'un quelconque des procédés selon l'invention d'identification automatique des contours d'au moins une portion d'un os prédéfini à partir d'une pluralité d'images décrits ci-dessus, comprenant les étapes du procédé selon l'invention de détermination automatique des paramètres initiaux d'une matrice de passage décrit ci-dessus ; - une étape de solidarisation de moyens de repérage audit élément de prothèse, de façon à fournir la position et l'orientation dudit élément de prothèse par rapport audit premier repère ; - une étape d'implantation par un praticien dudit élément de prothèse au niveau de ladite zone singulière dans une configuration spatiale déterminée au moins à partir de la représentation de la position et des contours dudit élément de prothèse et d'au moins ladite surface identifiée de ladite zone singulière dans ledit premier repère.

On décrit encore ici une méthode de guidage d'un outil chirurgical destiné à prélever au moins une partie d'une portion d'un os d'un patient comprenant :
- une étape d'obtention par une technique d'imagerie médicale d'une pluralité d'images représentant des coupes parallèles d'un volume de mesure comprenant ladite portion d'os ;
- une étape d'identification automatique des contours de ladite portion d'os à partir de ladite pluralité d'images comprenant les étapes de l'un quelconque des procédés d'identification automatique des contours d'au moins une portion d'un os prédéfini à partir d'une pluralité d'images décrits ci-dessus;
- une étape de solidarisation de moyens de repérage dans l'espace à ladite portion d'os, destinés à associer un premier repère à ladite portion d'os ;
- une étape de numérisation de ladite zone singulière ;
- une étape de recalage automatique dudit premier repère avec un deuxième repère associé auxdits contours de ladite portion d'os identifiés à partir de ladite pluralité d'images par la mise en oeuvre de l'un quelconque des procédés d'identification automatique des contours d'au moins une portion d'un os prédéfini à partir d'une pluralité d'images décrits ci-dessus, comprenant les étapes du procédé de détermination automatique des paramètres initiaux d'une matrice de passage décrit ci-dessus ;
- une étape de solidarisation de moyens de repérage audit outil chirurgical, de façon à fournir la position et l'orientation dudit outil chirurgical par rapport audit premier repère ; - une étape de guidage dudit outil au moins à partir de la représentation de la position et des contours dudit outil et de ladite portion d'os dans ledit premier repère.

### 5. Liste des figures

D'autres caractéristiques et avantages de l'invention apparaîtront plus clairement à la lecture de la description suivante d'un mode de réalisation de l'invention, donné à titre de simple exemple illustratif et non limitatif, et des dessins annexés parmi lesquels :
- la figure 1 représente un chirurgien lors d'une intervention de chirurgie prothétique de l'épaule utilisant une station de navigation hébergeant une application logicielle dédiée à la mise en oeuvre d'un mode de réalisation de procédés selon l'invention ;
- la figure 2 détaille une image affichée sur l'écran de la station de navigation présentée sur la figure 1 ;
- la figure 3 est une représentation des étapes d'un mode de réalisation du procédé selon l'invention d'identification automatique des contours d'une omoplate, sous forme de diagramme synoptique;
- les figures 4A à 4E représentent des images de différentes coupes transverses sur lesquelles sont repérées des formes étiquetées ; la figure 5 est un diagramme synoptique détaillant les étapes du procédé d'identification automatique de la surface glénoïdale selon l'invention ;
- la figure 6 détaille les étapes d'un mode de réalisation du procédé selon l'invention de détermination automatique des paramètres initiaux d'une matrice de passage, sous forme d'un diagramme synoptique ;
- la figure 7 est une représentation d'une interface de guidage du geste d'un chirurgien manipulant un outil chirurgical.

### 6. Description détaillée de l'invention

### 6.1 Rappel du principe de l'invention

Comme déjà indiqué, le principe de l'invention repose notamment sur la mise en oeuvre d'un étiquetage systématique des formes osseuses détectées de façon automatique au sein d'images obtenues par examen scanner ou IRM, faisant appel à une nomenclature osseuse appropriée et définie à l'avance, constituée d'un nombre limité d'étiquettes génériques distinctes. Cet étiquetage permet ensuite d'identifier le volume solide correspondant à l'os recherché de façon automatique.

L'invention permet également de ré-étiqueter certaines formes, si l'étiquette qui a été attribuée à cette forme est différente de celle associée à la plupart des formes se rattachant au même os que celui auquel se rattache cette forme.

### 6.2 Exemple de mode de réalisation de l'invention

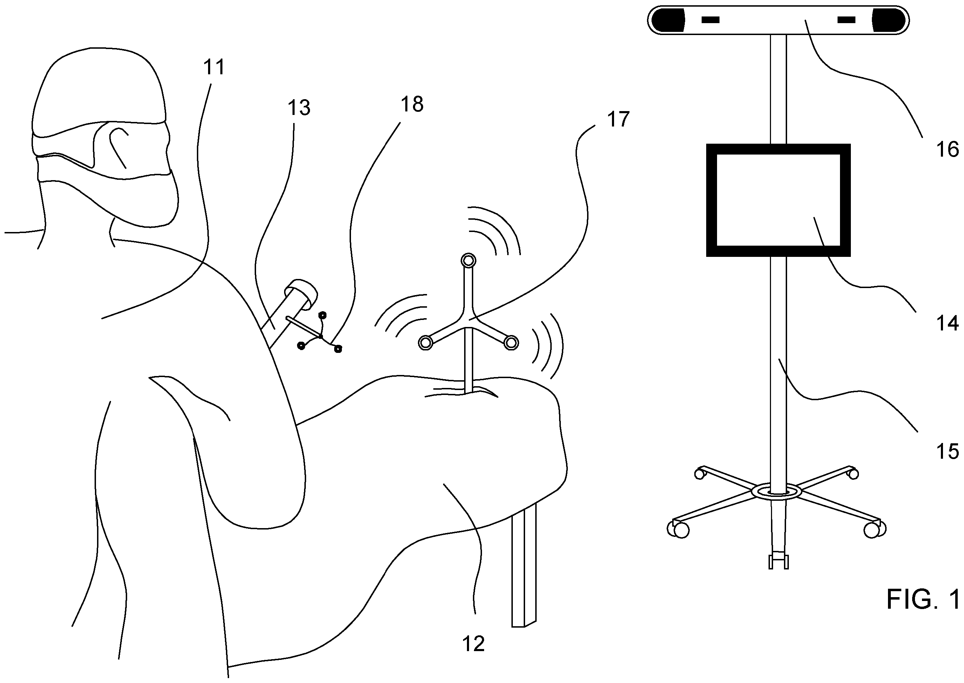

Sur la figure 1, on a représenté un chirurgien 11 se préparant à implanter un composant glénoïdien d'une prothèse d'épaule (non représenté sur la figure 1) au niveau de la surface glénoïdale de l'omoplate droite d'un patient 12, lors d'une intervention chirurgicale orthopédique de l'épaule. Ce chirurgien 11 tout en déplaçant le composant prothétique glénoïdien au bout du manche 13, peut observer en temps réel la position de ce composant par rapport à la surface de l'omoplate sur l'écran 14 d'une station de navigation 15, équipée d'un localisateur optique 16.

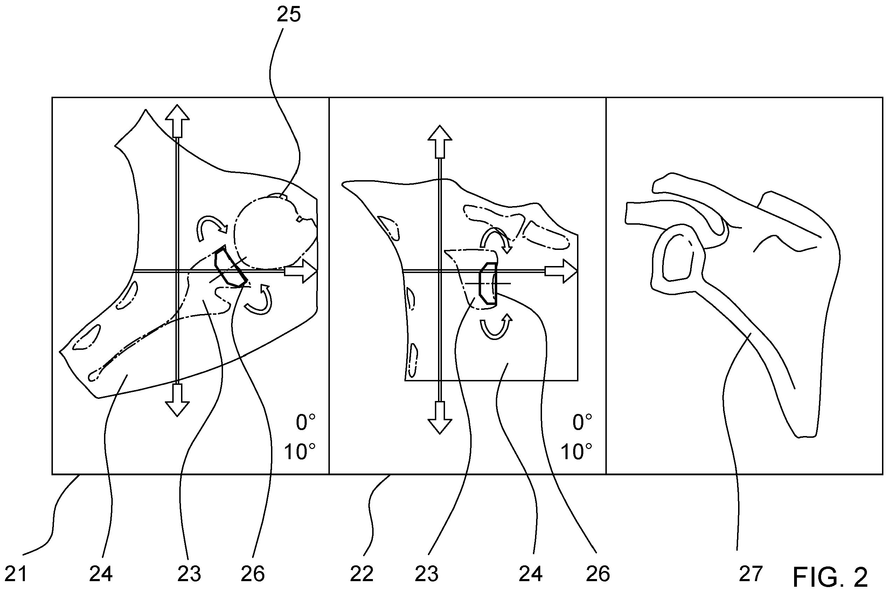

La figure 2 est une vue de détail d'un triptyque affiché sur l'écran 14. On distingue à gauche (cadre 21) et au centre (cadre 22) de ce triptyque une même coupe 2D de la région de l'articulation de l'épaule suivant un plan transverse, sur lesquelles apparaissent en surbrillance le contour de la surface glénoïdale 23, et plus généralement de la surface 24 de l'omoplate, le contour de la tête humérale 25 et un contour du composant prothétique glénoïdien 26. Dans le cadre 21, le contour 26 est représenté dans sa position réelle par rapport à l'omoplate. Le cadre 22 permet au chirurgien de visualiser une position théorique optimale d'implantation du composant prothétique glénoïdien. Par ailleurs, les valeurs de l'angle de rétroversion et d'inclinaison, en l'occurrence respectivement 0 et 10°, s'affichent sur l'écran 14. D'autres informations telles que le plan de référence de l'omoplate, le plan de référence de la surface glénoïdale, l'angle de version de la surface glénoïdale, et le capital osseux de la glène sont également accessibles automatiquement grâce à la mise en oeuvre de l'invention. La restitution de ces informations en regard du contour géométrique 26 du composant glénoïdien a permis au chirurgien d'optimiser la forme de cet élément prothétique et de simuler la pose de cet élément avant l'opération.

Les contours 27 de l'omoplate sont représentés en 3D à droite de la figure 2. Ces contours ont été préalablement modélisés, via une première application logicielle, dans une phase pré-opératoire grâce au procédé d'identification automatique de contours d'une portion d'os selon l'invention, paramétré pour la région de l'épaule. Ils ont été obtenus à partir d'une centaine d'images de coupes 2D transverses d'un volume de mesure centré sur l'articulation de l'épaule, après que le patient se soit rendu dans un service de radiologie pour réaliser un scanner. Les coupes 2D présentent une résolution sensiblement égale à 0,5 mm dans ce mode de réalisation de l'invention.

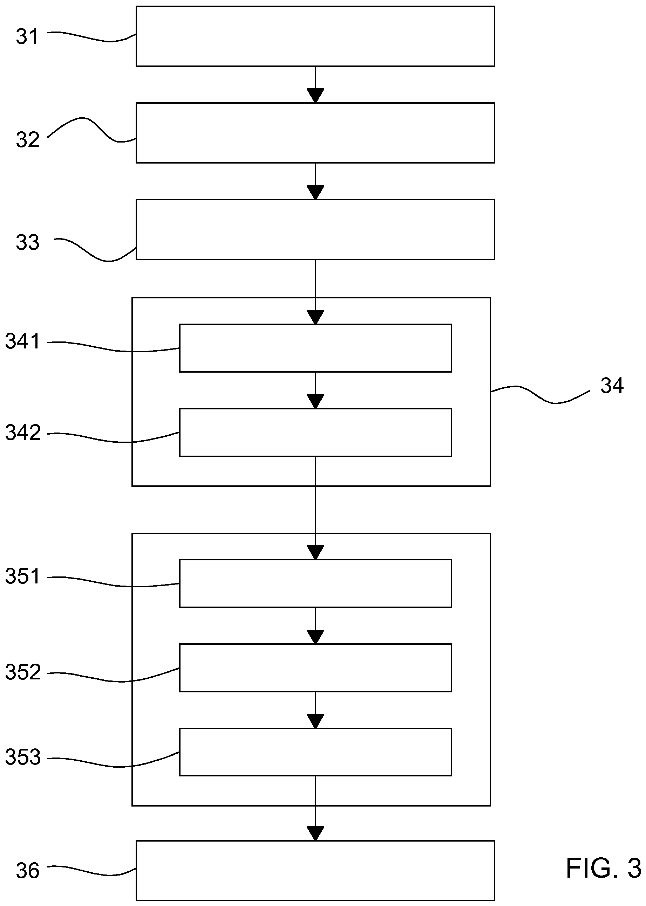

La figure 3 détaille sous forme de diagramme synoptique les étapes d'un mode de réalisation du procédé d'identification automatique de contours mis en oeuvre pour obtenir les contours 26.

Dans une première étape 31, les images des coupes 2D sont filtrées de façon à ne conserver que les points élémentaires de l'image dont la densité correspond à celle d'un tissu osseux. À cet effet, on compare par exemple l'intensité de chaque point de l'image, exprimé par exemple en unité de densité Hounsfield, avec une intensité de référence, fixée dans ce mode de réalisation de l'invention à 250 Hounsfield, et on écarte les points de l'image dont l'intensité est inférieure à cette intensité de référence. Dans une variante de ce mode de réalisation, il peut également être envisagé d'écarter les points de l'image dont l'intensité est supérieure à 300 Hounsfield, caractéristiques de corps dont l'intensité est supérieure à la densité osseuse maximale connue.

Dans une deuxième étape 32, on applique des filtres morphologiques de formulation connue aux images filtrées dans le but de détecter des formes de contour fermé, ou composantes connexes, de tailles conséquentes au sein de chaque image. Cette opération de segmentation permet de supprimer de l'image les composantes connexes de taille réduite.

L'intérieur des contours fermés est également comblé dans une étape suivante 33, de sorte à faire disparaître de l'image les composantes connexes qui s'apparentent à des corticales internes.

Une étiquette sélectionnée au sein d'une nomenclature osseuse prédéfinie est par la suite associée à chacune des formes de contour fermé subsistant dans l'image, dans une étape 34. Cette nomenclature regroupe les étiquettes suivantes dans ce mode de réalisation de l'invention consacré à l'identification d'os de l'épaule : "os long", "os plat", "autres os" et "table d'examen".

Lors de l'étape 34, les valeurs de trois paramètres géométriques adimensionnels propres à chaque forme, formant un triplet, sont comparées une à une, dans une étape préliminaire 341, aux valeurs prédéfinis correspondantes de chaque triplet de référence d'une base de données.

Cette base de données préétablie a été constituée à partir de relevés anatomiques effectués sur une grande population de patients. Elle présente une table de corrélation faisant correspondre à chaque triplet de référence une étiquette prédéfinie de la nomenclature osseuse.

Si le triplet propre à une forme est sensiblement semblable à un triplet de référence, ou en d'autres termes que chacun des paramètres diffère seulement de quelques pourcents par rapport aux valeurs d'un des triplets de référence, on affecte à cette forme l'étiquette attachée à ce triplet de référence, et par exemple l'étiquette os long.

Les paramètres géométriques retenus dans ce mode de réalisation sont:
- l'élongation, qui se définit comme le rapport du diamètre du cercle inscrit du contour sur le diamètre du cercle circonscrit au contour de la forme ;
- le rectangle englobant qui se définit comme le rapport de la longueur sur la largeur d'un rectangle circonscrit dont les bords sont tangents aux contours de la forme ;
- la compacité qui se définit comme le rapport de la longueur du contour de la forme sur la surface interne au contour.

Dans des variantes de ce mode de réalisation, deux, quatre, cinq paramètres géométriques adimensionnels propres à chaque forme, ou plus, peuvent être comparés à des paires, quadruplets, quintuplets prédéfinis rassemblés dans des bases de données de référence, et/ou un moment d'inertie normalisé de la forme peut constituer l'un de ces paramètres géométriques, par exemple.

Les inventeurs ont constaté que cette étape 341 est souvent suffisante pour identifier des formes de la table d'examen, du fait de leurs élongations importantes, et des côtes. Dans ce mode de réalisation de l'invention, on attribue L'étiquette "autre os" aux formes reconnues comme celles de côtes.

Dans une étape suivante 342, une étiquette va être affectée de façon automatique à chacune des formes restant sans étiquette à l'issue de l'étape 341.

Lors de cette étape 342, on détermine pour chacune des formes sans étiquette les 32 premiers descripteurs invariants normalisés de Fourier de leur contour, ou en d'autres termes les trente deux premiers coefficients de la transformée de Fourier de leur contour, normalisés pour les rendre indépendants des facteurs d'échelle et rendus invariants au regard de la rotation et de la translation. On note que 32 descripteurs de Fourier sont généralement suffisants pour permettent de rendre compte de la complexité des contours d'un os long, par exemple.

Dans des variantes, il peut être envisagé de déterminer les 8, 16 ou 64 premiers descripteurs de Fourier de ces contours, selon la complexité des contours à traiter. Un vecteur invariant normalisé est ensuite formé à partir de ces 32 premiers descripteurs, puis on applique un processus de reconnaissance de forme à ce vecteur. Plus précisément, la distance de ce vecteur avec chacun des vecteurs invariants normalisés d'une bibliothèque de formes de contour d'os préétablie est évaluée, et on affecte à la forme traitée l'étiquette attachée au vecteur de la bibliothèque minimisant cette distance, au sens de la norme euclidienne par exemple.

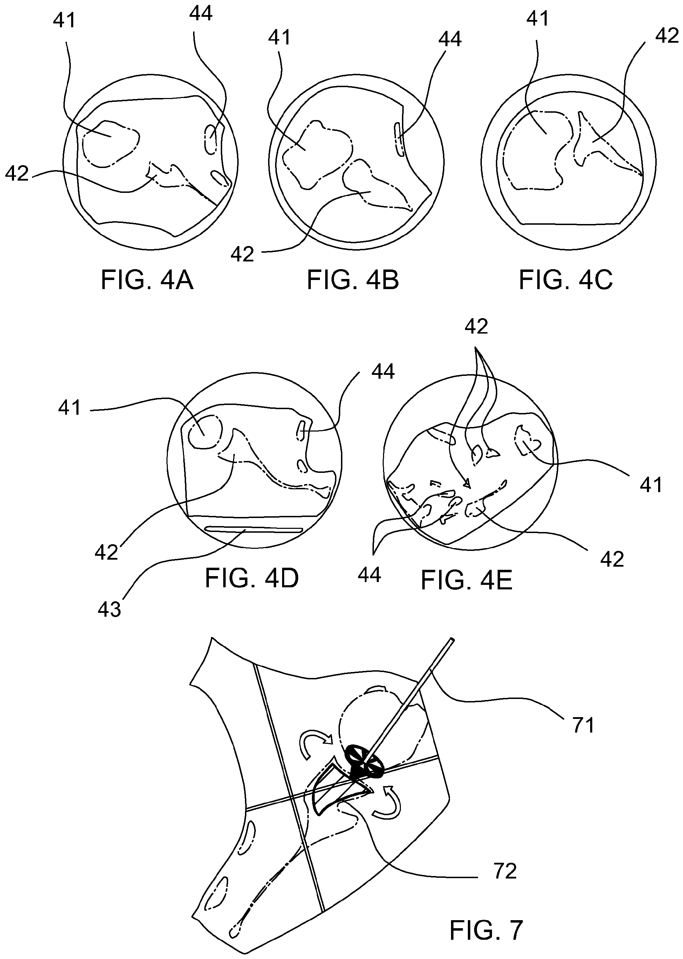

Sur les figures 4A à 4E sont représentés des résultats d'association d'étiquette à des formes de contour fermé sur différentes coupes parallèles, obtenus à l'issue de l'étape 34. On distingue sur ces figures des formes 41 associées à l'étiquette "os long" et correspondant à l'humérus, des formes 42 associées à l'étiquette "os plat" et correspondant à l'omoplate, des formes 43 associées à l'étiquette "table d'examen" et des formes 44 associées à l'étiquette "autres os", comprenant des coupes de côtes, ...

Dans une dernière étape 35, on identifie les contours de l'omoplate en classifiant les formes par groupe de formes (étape 351), par exemple en appliquant une méthode de classification hiérarchique ou stochastique connue en soi. Plus précisément, on trie les formes par groupe appartenant à la surface d'un même volume tridimensionnel isolé dans l'espace, ou en d'autres termes on reconstruit des volumes solides 3D à partir des formes obtenues à l'étape 32.

Dans ce mode de réalisation particulier de l'invention, on procède en pratique de la façon suivante pour classifier les formes :
On recherche sur deux images correspondant à des couches parallèles adjacentes, des formes dont les contours se superposent sensiblement lorsqu'on projette ces images l'une sur l'autre. On vérifie notamment si leurs centres de gravité et leurs contours coïncident sensiblement, par exemple du fait de leur proximité ou du fait qu'ils se coupent plusieurs fois, ce qui permet de reconstruire pas-à-pas chacun des volumes solides, c'est-à-dire des os du volume de mesure scanographié. Dans une étape 352, on détermine ensuite le groupe de formes, dit groupe de formes cible, pour lequel l'étiquette "os plat", correspondant à l'omoplate, est majoritairement associée aux formes d u groupe. Il convient de noter que dans le cadre de l'invention, la notion de "majorité" est entendue dans une acception large. Il peut par exemple s'ag ir d'une estimation basée au moins en partie sur un critère statistique.

On a ainsi identifié de façon automatique les contours de l'omoplate, qui sont les contours des formes du groupe de formes cible, et que l'on sélectionne (étape 353).

Une étape 36 ultérieure est également prévue pour prendre en compte des erreurs d'étiquetage au sein des formes du groupe de formes cible. Plus précisément, on remplace les étiquettes des formes de ce groupe différentes d"'os plat" par l'étiquette "os plat" correspondant à l'omoplate.

Les données des contours de l'omoplate identifiés ont ensuite été traitées, lors de la phase pré-opératoire, par une deuxième application logicielle qui permet d'extraire automatiquement une représentation tridimensionnelle de la surface glénoïdale, à partir de sa "signature de forme".

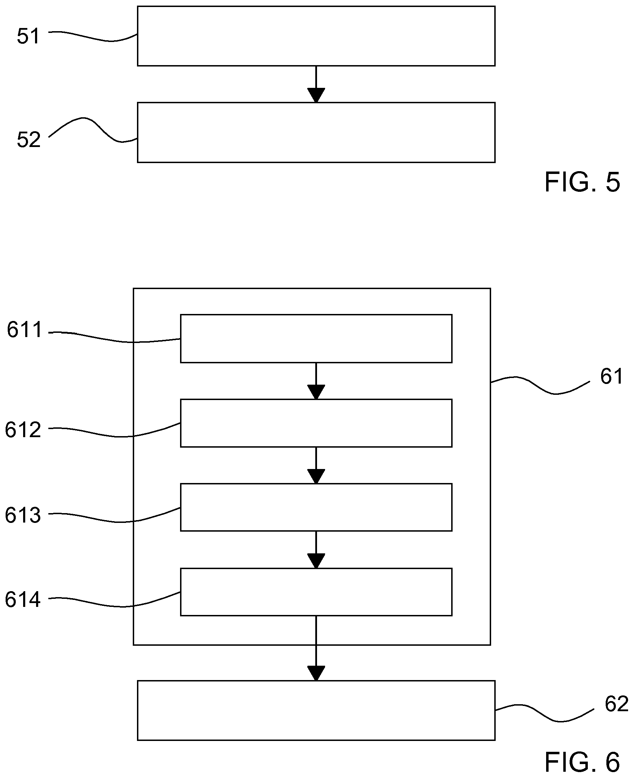

Les étapes permettant d'identifier cette surface sont détaillées sous forme de diagramme synoptique sur la figure 5.

Dans une étape 51, la position tridimensionnelle d'un point anatomique spécifique de l'omoplate proche de la surface glénoïdale, est repéré sur la base du modèle numérique de l'omoplate reconstituée à partir de ses contours.

Dans une étape 52, la surface glénoïdale est recherchée au sein d'une calotte sphérique centrée autour de ce point, de rayon et d'épaisseur fixés en fonction de la distance généralement constatée entre ce point spécifique et la surface glénoïdale, et en fonction d'une estimation théorique de la taille de la surface glénoïdale.

Le plan médian au sens des moindres carrés, l'angle de version et l'ang le d'inclinaison de la glène sont ensuite déterminés par une routine de calcul usuelle. Une procédure analogue est utilisée pour obtenir une modélisation numérique en 3D de la surface de l'acromion et de la surface du processus coracoïde.

Avant d'introduire le composant 26 dans l'incision dans le but de l'implanter dans une configuration spatiale prédéterminée, le chirurgien a pratiqué les actes suivants, ne demandant que quelques minutes, afin d'obtenir l'image de la figure 2:
- il a solidarisé à l'aide d'une pince un tripode portant un corps rigide 17, ou "tracker" de référence, incluant des marqueurs qui renvoient de façon passive le rayonnement infrarouge vers le localisateur optique 16, de façon à associer un repère spatial à l'omoplate. On note que dans une variante, ce tracker peut être un tracker actif équipé de diodes infra-rouges, ou encore un capteur électromagnétique si un localisateur magnétique est utilisé ;
- il a palpé la surface glénoïdale de façon à permettre le calcul automatique d'une matrice de passage initiale entre le repère spatial associé à l'omoplate du patient et un repère associé à la modélisation numérique de l'omoplate 26;
- il a palpé, à titre subsidiaire, la surface de l'acromion et la surface du processus coracoïde pour affiner les paramètres de cette matrice de passage initiale.

Un tripode 18, portant un corps rigide, est également solidarisé sur le manche 13, ce qui permet de repérer la position du composant prothétique 26 dans l'espace. La position relative du composant 26 par rapport au tripode 18 a été obtenue par une technique de calibrage connue.

Le recalage entre le repère spatial de l'omoplate du patient, encore appelé par la suite premier repère, avec un deuxième repère associé à la modélisation numérique de l'omoplate est réalisé automatiquement par une application logicielle, permettant d'évaluer une matrice de passage entre ces deux repères.

Comme on peut le voir sur le diagramme synoptique de la figure 6, les paramètres de cette matrice de passage sont déterminés en deux phases, représentées en traits pointillés sur la figure 6. Des paramètres initiaux permettant d'aboutir à cette matrice sont déterminés dans une première phase 61.

Dans une deuxième phase 62, un algorithme de recalage itératif connu est appliqué à une matrice formée à partir de ces paramètres initiaux, encore appelée par abus de langage matrice de passage initiale, ce qui permet d'obtenir les paramètres d'une matrice de passage affinée permettant de minimiser la distance entre la surface des zones palpées de l'omoplate et la modélisation numérique cette surface, obtenue conformément au procédé d'identification de surface présenté en référence à la figure 5.

Lors de la phase 61, on calcule dans une étape 611 le barycentre de la modélisation numérique en 3D de la surface glénoïdale, identifiée via les étapes 51 et 52. On détermine également dans cette étape 611, le vecteur normal au plan médian de cette surface glénoïdale modélisée, et orienté vers l'extérieur de l'omoplate. Ce calcul est effectué en utilisant une approximation robuste au sens des moindres carrés des points de la surface par un plan visant à minimiser la somme des carrés des distances entre les points de la surface et le plan recherché, tout en éliminant les données aberrantes dont l'écart au plan est supérieur à trois fois la variance des écarts pour l'ensemble des points.

Dans une étape suivante 612, on évalue des paramètres de recalage permettant de faire coïncider respectivement les coordonnées du barycentre obtenu à l'étape 611 avec les coordonnées du barycentre de la surface glénoïdale numérisée par palpage, et les coordonnées du vecteur obtenu à l'étape 611 avec celles du vecteur normal au plan médian de la surface glénoïdale numérisée, d'orientation connue grâce aux palpeurs. La rotation autour de ce vecteur normal reste indéterminée à ce stade.

On transcrit ensuite les paramètres de recalage en paramètres initiaux de la matrice de passage, dans une étape 613.

Ces paramètres initiaux sont affinés dans une étape suivante 614, en minimisant la distance entre les surfaces théoriques de l'acromion et du processus coracoïde, calculées automatiquement à partir des images scanner, et les coordonnées résultant de la numérisation par palpage de la surface de l'acromion et de la surface du processus coracoïde, en effectuant des rotations autour de l'axe défini par le vecteur normal au plan médian de la surface glénoïdale de façon à minimiser la distance entre les points palpés et les surfaces modélisées. À cet effet, l'algorithme de Levenberg-Marquardt peut par exemple être appliqué.

### 6.3 Autres caractéristiques optionnelles et avantages de l'invention

Dans des variantes du mode de réalisation de l'invention détaillé ci-dessus, il peut également être prévu de guider le geste d'un chirurgien lorsqu'il manipule une fraise chirurgicale, un foret chirurgical ou tout autre outil chirurgical, en affichant sur l'écran 14 la position et l'angle de l'outil 71 qu'il tient en main par rapport à l'os foré 72, par exemple sous la forme illustrée figure 7. Ce guidage permis par le procédé de guidage selon l'invention nécessite de solidariser un repère à l'outil. Il permet de sécuriser le geste du chirurgien au cours d'une opération délicate, et par exemple au cours d'une opération d'arthroplastie de l'épaule.

Dans une autre variante, il peut être envisagé, sans sortir du cadre de l'invention, que la nomenclature osseuse puisse également comprendre au moins une des étiquettes suivantes : os court, os allongé, os rayonné, os arqué, os papyracé, os pneumatique, os sésamoïde.

## Revendications

1. Procédé d'identification automatique des contours d'au moins une portion d'un os prédéfini de l'épaule à partir d'une pluralité d'images représentant des coupes parallèles d'un volume de mesure comprenant ladite portion d'os, obtenues par une technique d'imagerie médicale, telle que la tomodensitométrie ou l'imagerie par résonance magnétique,
**caractérisé en ce qu'**il comprend :
(a) une étape de filtrage desdites images comprenant une étape de comparaison, pour chacune desdites images, de l'intensité d'au moins un point élémentaire de ladite image avec une intensité de référence, de façon à obtenir une image filtrée comprenant lesdits points élémentaires de l'image dont la densité correspond à celle d'un tissu osseux ;
(b) une étape d'obtention d'au moins une forme de contour fermé dans au moins une desdites images filtrées par application d'au moins un filtre morphologique auxdites images filtrées ;
(c) une étape d'association à chacune desdites formes d'une étiquette sélectionnée au sein d'une nomenclature prédéfinie, ladite nomenclature prédéfinie comprenant les termes os long, os plat, autres os et table d'opération et ladite étape d'association comprenant, pour chacune desdites formes, les étapes suivantes dans cet ordre :
- une étape de comparaison d'un n-uplet de données adimensionnelles avec au moins un n-uplet prédéfini, n'étant supérieur ou égal à 2, lesdites données appartenant au groupe comprenant au moins l'élongation, la compacité, le rectangle englobant et les moments d'inertie normalisés de ladite forme de contour fermé, le rectangle englobant étant défini comme le rapport de la longueur sur la largeur d'un rectangle circonscrit dont les bords sont tangents aux contours de ladite forme ;
- dans le cas où ledit ensemble de n valeurs est sensiblement semblable à l'un desdits n-uplets prédéfinis, une étape d'affectation à ladite forme d'une étiquette attachée audit n-uplet prédéfini, l'ensemble étant dit sensiblement semblable lorsque chacune des n valeurs diffère seulement de quelques pourcents par rapport aux valeurs d'un desdits n-uplets prédéfinis ; et
- une étape d'affectation à chacune des formes restant sans étiquette à l'issue de ladite étape d'affectation à ladite forme d'une étiquette attachée audit n-uplet prédéfini, d'une étiquette attachée à un vecteur invariant normalisé prédéfini sélectionné parmi une pluralité de vecteurs invariants normalisés prédéfinis, minimisant la distance avec un vecteur invariant normalisé formé d'un nombre prédéterminé de coefficients normalisés représentatifs des coordonnées dudit contour dans l'espace de Fourier ;
(d) une étape d'identification desdits contours de ladite portion d'os comprenant:
- une étape de groupement desdites formes de sorte à former au moins un groupe de formes délimitant un volume commun isolé dans l'espace ;
- une étape de détermination parmi lesdits groupes de forme, d'un groupe de formes cible pour lequel l'étiquette correspondant audit os est majoritairement associée aux formes du groupe ;
- une étape de sélection des formes dudit groupe de formes cible.

2. Procédé d'identification selon la revendication 1, **caractérisé en ce qu'**il comprend en outre une étape de remplacement des étiquettes des formes dudit groupe cible sélectionnées ne correspondant pas audit os prédéfini par ladite étiquette correspondant audit os prédéfini.

3. Procédé d'identification selon l'une quelconque des revendications 1 et 2, **caractérisé en ce que** les étiquettes attachées aux vecteurs invariants normalisés prédéfinis appartiennent au groupe comprenant au moins :
- os long ;
- os court;
- os allongé
- os rayonné
- os arqué ;
- os papyracé ;
- os pneumatique ;
- os sésamoïde.

4. Procédé d'identification selon la revendication 3, **caractérisé en ce que** ledit nombre prédéterminé de coefficients est supérieur ou égal à 8.

5. Procédé d'identification selon l'une quelconque des revendications 1 à 4 dans lequel ledit os prédéfini est une omoplate ou un humérus.

6. Procédé d'identification automatique de la surface d'au moins une zone singulière appartenant au groupe comprenant :
- une saillie ;
- une cavité ;
- une zone sensiblement plane,
sur une portion d'un os prédéfini dont les contours ont été obtenus conformément à l'un quelconque des procédés selon l'une des revendications 1 à 5, **caractérisé en ce qu'**il comprend :
- une étape d'obtention de la position tridimensionnelle d'au moins un point anatomique spécifique propre à ladite surface de ladite portion d'un os prédéfini à partir desdits contours ;
- une étape d'identification de ladite surface de ladite zone singulière dans une calotte sphérique centrée autour dudit point, de rayon et d'épaisseur prédéfinis et fonction de la distance dudit point à ladite zone singulière et d'une estimation de la taille de ladite zone singulière.

7. Procédé d'identification selon la revendication 6, **caractérisé en ce que** ledit os prédéfini étant une omoplate, ladite zone singulière appartient au groupe comprenant :
- acromion ;
- surface glénoïdale ;
- processus coracoïde.

8. Procédé de détermination automatique des paramètres initiaux d'une matrice de passage entre un premier repère associé à au moins une portion d'un os d'un patient et un deuxième repère associé aux contours de ladite portion d'os identifiés sur une pluralité d'images obtenues par une technique d'imagerie médicale représentant des coupes d'un volume de mesure comprenant ladite portion d'os, **caractérisé en ce qu'**il comprend :
- une étape d'identification automatique de la surface d'une zone singulière sur lesdites images comprenant les étapes du procédé selon la revendication 6 ;
- une étape de détermination du barycentre de ladite zone singulière et du vecteur normal au plan médian de ladite surface de ladite zone singulière orienté vers l'extérieur, ledit plan médian étant une approximation au sens des moindres carrés de la surface de la zone singulière ;
- une étape d'obtention de paramètres de recalage résultant de la coïncidence dudit barycentre et dudit vecteur normal déterminés avec le barycentre de ladite zone singulière numérisée à l'aide d'un laser, à l'aide d'une sonde échographique ou par palpage sur ladite portion d'os et un vecteur normal au plan médian de la surface de ladite zone singulière numérisée, orienté vers l'extérieur ;
- une étape de formation desdits paramètres initiaux à partir desdits paramètres de recalage.

9. Procédé de simulation de la pose d'un élément d'une prothèse au niveau d'une zone singulière d'une portion d'un os prédéfini de l'épaule comprenant :
- une étape d'identification automatique de la surface de ladite zone singulière sur des images de ladite portion d'un os prédéfini de l'épaule obtenues par une technique d'imagerie médicale comprenant les étapes du procédé selon la revendication 6 ;
- une étape de détermination d'une première information comprenant le plan de référence de ladite surface, et/ou l'angle de version et/ou l'angle d'inclinaison associés à ladite surface ;
- une étape de détermination d'une deuxième information représentative du capital osseux associé à ladite surface, à partir de la donnée de ladite surface et de la donnée des contours de ladite portion d'os obtenus conformément au procédé d'identification selon l'une des revendications 1 à 5 ;
- une étape de restitution desdites première et deuxième informations en regard d'au moins un paramètre géométrique de l'élément de prothèse.

10. Produit programme d'ordinateur téléchargeable depuis un réseau de communication et/ou enregistré sur un support lisible par ordinateur et/ou exécutable par un processeur **caractérisé en ce qu'**il comprend des instructions de code de programme pour l'exécution des étapes d'un quelconque des procédés d'identification automatique des contours d'au moins une portion d'un os prédéfini selon l'une des revendications 1 à 5 et/ou des étapes d'un quelconque des procédés d'identification automatique d'au moins une zone singulière d'au moins une portion d'un os prédéfini selon l'une des revendications 6 ou 7 et/ou des étapes du procédé de détermination paramètres initiaux d'une matrice de passage selon la revendication 8 et/ou des étapes du procédé de simulation selon la revendication 9, lorsque ledit programme est exécuté par un ordinateur.

## Patentansprüche

1. Verfahren zur automatischen Identifikation der Konturen mindestens eines Teils eines vordefinierten Knochens der Schulter aus einer Mehrzahl von Bildern, die parallele Schnitte eines den Teil des Knochens umfassenden Messvolumens darstellen, die durch eine Technik der medizinischen Bildgebung, wie die Computertomografie oder die Bildgebung durch Magnetfeldresonanz, erhalten werden, ,
**dadurch gekennzeichnet, dass** es umfasst:
(a) einen Schritt des Filterns der Bilder, der für jedes der Bilder einen Schritt des Vergleichens der Intensität mindestens eines elementaren Punktes des Bildes mit einer Referenzidentität, um ein filtriertes Bild zu erhalten, das die elementaren Punkte des Bildes, deren Dichte derjenigen eines Knochengewebes entspricht, umfasst;
(b) mindestens einen Schritt des Erhaltens mindestens einer geschlossenen Konturform in mindestens einem der filtrierten Bilder durch Anwendung mindestens eines morphologischen Filters auf die filtrierten Bilder;
(c) einen Schritt des Zuordnen zu jeder der Formen eines ausgewählten Kennzeichens in einer vordefinierten Nomenklatur, wobei die vordefinierte Nomenklatur die Begriffe langer Knochen, flacher Knochen, andere Knochen und Operationstisch umfasst,
und der Schritt des Zuordnens für jede der Formen die folgenden Schritte in dieser Reihenfolge umfasst:
- einen Schritt des Vergleichens eines n-Tupels von eindimensionalen Daten mit mindestens einem vordefinierten n-Tupel, das nicht größer als oder gleich 2 ist, wobei die Daten zur Gruppe gehören, die mindestens die Längung, die Kompaktheit, das begrenzende Rechteck, die normalisierten Trägheitsmomente der geschlossenen Konturform, wobei das begrenzende Rechteck definiert ist als das Verhältnis der Länge zur Breite eines umschriebenen Rechtecks, dessen Ränder Tangenten an die Konturen der Form sind;
- in dem Fall, in dem die Gesamtheit von n Werten im Wesentlichen gleich einem der vordefinierten n-Tupel ist, einen Schritt des Zuweisens eines dem vordefinierten n-Tupel anhängenden Kennzeichens zu der Form, wobei die Gesamtheit im Wesentlichen als gleich genannt wird, wenn jeder der n Werte nur um einige Prozente in Bezug auf die Werte eines der vordefinierten n-Tupel differiert; und
- einen Schritt des Zuweisens eines Kennzeichens, das einem vordefinierten normalisierten invarianten Vektor anhängt, der aus einer Mehrzahl von vordefinierten normalisierten invarianten Vektor ausgewählt ist, zu jeder der Formen, die nach dem Ende des Schritts des Zuweisens eines dem vordefinierten n-Tupel anhängenden Kennzeichens zu der Form ohne Kennzeichen verblieben sind, wobei die Distanz zu einem normalisierten invarianten Vektor minimiert wird, der aus einer vorbestimmten Anzahl von normalisierten Koeffizienten, die für die Koordinaten der Kontur im Fourierraum repräsentativ sind, gebildet wird;
(d) einen Schritt des Identifizierens der Konturen des Knochenteils, umfassend:
- einen Schritt des Gruppierens der Formen derart, dass mindestens eine Formengruppe gebildet wird, die ein isoliertes gemeinsames Volumen im Raum begrenzt;
- einen Schritt des Bestimmens einer Zielformengruppe aus den Formengruppen, für die das dem Knochen entsprechende Kennzeichen mehrheitlich den Formen der Gruppe zugeordnet ist;
- einen Schritt des Auswählens der Formen der Zielformengruppe.

2. Verfahren zur Identifikation nach Anspruch 1, **dadurch gekennzeichnet, dass** es außerdem einen Schritt des Ersetzens der ausgewählten Kennzeichen der Formen der Zielgruppe, die nicht dem genannten vordefinierten Knochen entsprechen, durch das dem vordefinierten Knochen entsprechende Kennzeichen umfasst.

3. Verfahren zur Identifikation nach einem beliebigen der Ansprüche 1 und 2, **dadurch gekennzeichnet, dass** die den vordefinierten normalisierten invarianten Vektoren anhängenden Kennzeichen zu der Gruppe gehören, die mindestens umfassen:
- langer Knochen;
- kurzer Knochen;
- langgestreckter Knochen
- Radiusknochen
- gebogene Knochen
- Papyrus-Knochen
- luftgefüllte Knochen
- Sesambeine.

4. Verfahren zur Identifikation nach Anspruch 3, **dadurch gekennzeichnet, dass** die vorbestimmte Anzahl von Koeffizienten größer oder gleich 8 ist.

5. Verfahren zur Identifikation nach einem beliebigen der Ansprüche 1 bis 4, bei dem der vordefinierte Knochen ein Schulterblatt oder Humerus ist.

6. Verfahren zur automatischen Identifikation der Fläche mindestens einer singulären Zone, die zu der Gruppe gehört, die umfasst:
- einen Vorsprung;
- eine Kavität;
- eine im wesentlichen ebene Zone
auf einem Bereich eines vordefinierten Knochens, dessen Konturen nach einem beliebigen der Verfahren nach einem der Ansprüche 1 bis 5 erhalten wurden, **dadurch gekennzeichnet, dass** es umfasst:
- einen Schritt des Erhaltens der dreidimensionalen Position von mindestens einem spezifischen anatomischen Punkt, der der Fläche des Teils eines vorbestimmten Knochens eigen ist, aus den Konturen;
- einen Schritt des Identifizierens der Fläche der singulären Zone in einer Kugelkalotte, die um den Punkt zentriert ist, von vordefiniertem Radius und vordefinierter Ddicke und abhängig von der Entfernung des Punktes zu der singulären Zone und einer Schätzung der Größe der singulären Zone.

7. Verfahren zum Identifizieren nach Anspruch 6, **dadurch gekennzeichnet, dass** der vordefinierte Knochen ein Schulterblatt ist, wobei die singuläre Zone zu der Gruppe gehört, die umfasst:
- Acromion;
- Glenoidfläche;
- Rabenschnabelfortsatz (processus coracoides).

8. Verfahren zur automatischen Bestimmung von Anfangsparametern einer Übergangsmatrix zwischen einem ersten Bezugspunkt, der zumindest einem Teil eines Knochens eines Patienten zugeordnet ist, und einem zweiten Bezugspunkt, der den Konturen des Teils des auf einer Mehrzahl von Bildern identifizierten Knochens zugeordnet ist, die durch eine Technik der medizinischen Bildgebung erhalten werden, die Schnitte eines den Teil des Knochens umfassenden Messvolumens darstellen, **dadurch gekennzeichnet, dass** es umfasst:
- einen Schritt des automatischen Identifizierens der Fläche einer singulären Zone auf den Bildern, der die Schritte des Verfahrens nach Anspruch 6 umfasst;
- einen Schritt des Bestimmens des Schwerpunktes der singulären Zone und des Normalenvektors zur Mittelebene der Fläche der singulären Zone, der nach außen gerichtet ist, wobei die Mittelebene eine Näherung im Sinne der kleinsten Quadrate der Fläche der singulären Zone ist;
- einen Schritt des Erhaltens von Bildregistrierungsparameter, die aus der Koinzidenz des Schwerpunktes und des Normalenvektors und die mit dem Schwerpunkt der singulären Zone, die mithilfe eines Lasers, mithilfe einer Schallsonde oder durch Abtastung über den Teil des Knochens digitalisiert wird, und einem Normalenvektor zur Mittelebene der Fläche der digitalisierten singulären Zone, der nach außen gerichtet ist, bestimmt werden;
- einen Schritt des Bildens der Anfangsparameter aus den Bildregistrierungsparametern.

9. Verfahren zum Simulieren der Anordnung eines Elementes einer Prothese an einer singulären Zone eines Teils eines vordefinierten Knochens der Schulter, umfassend:
- einen Schritt des automatischen Identifizierens der Fläche der singulären Zone auf Bildern des Teils eines vordefinierten Knochens der Schulter, die durch eine Technik der medizinischen Bildgebung erhalten werden, der die Schritte des Verfahrens nach Anspruch 6 umfasst;
- einen Schritt des Bestimmens einer ersten Information, die die Referenzebene der Fläche und/oder den Versionswinkel und/oder den Neigungswinkel, die der Fläche zugeordnet sind, umfasst;
- einen Schritt des Bestimmens einer zweiten Information, die repräsentativ für die der Fläche zugeordneten Knochenmasse ist, aus den Daten der Fläche und den Daten der Konturen des Teils des Knochens, die entsprechend dem Verfahren zum Identifizieren nach einem der Ansprüche 1 bis 5 erhalten werden;
- einen Schritt des Wiederherstellens der ersten und zweiten Information hinsichtlich mindestens eines geometrischen Parameters des Prothesenelementes.

10. Rechnerprogrammprodukt, das aus einem Kommunikationsnetz herunterladbar ist und/oder auf einem von dem Rechner lesbaren Datenträger registriert ist und/oder durch einen Prozessor durchführbar ist, **dadurch gekennzeichnet, dass** es Softwarebefehle für die Durchführung der Schritte eines beliebigen der Verfahren zum automatischen Identifizieren von Konturen mindestens eines Teils eines vordefinierten Knochens nach einem der Ansprüche 1 bis 5 und/oder der Schritte eines beliebigen der Verfahren zum automatischen Identifizieren mindestens einer singulären Zone mindestens eines Teils eines vordefinierten Knochens nach einem der Ansprüche 6 oder 7 und/oder Schritte des Verfahrens zum Bestimmen von Anfangsparametern einer Übergangsmatrix nach Anspruch 8 und/oder Schritte des Verfahrens zum Simulieren nach Anspruch 9 umfasst, wenn das Programm von einem Rechner durchgeführt wird.

## Claims

1. A method for the automatic identification of the contours of at least one portion of a predefined bone of the shoulder from a plurality of images representing parallel sections of a measuring volume comprising said bone portion, obtained by a medical imaging technique, such as computed tomography or magnetic resonance imaging, **characterized in that** it includes:
(a) a step for filtering of said images including a step for comparing, for each of said images, the intensity of at least one elementary point of said image with a reference intensity, so as to obtain a filtered image including said elementary points of the image whose density corresponds to that of a bone tissue;
(b) a step for obtaining at least one closed contour shape in at least one of said filtered images by applying at least one morphological filter to said filtered images;
(c) a step for associating, with each of said shapes, a tag selected from within a predefined nomenclature, said predefined nomenclature including the terms long bone, flat bone, other bone and operating table in the case and said associating step including, for each of said shapes, the following steps in this order:
- a step for comparing an n-tuple of dimensionless data with at least one predefined n-tuple, n being greater than or equal to 2, said data belonging to the group including at least the elongation, the compactness, the bounding box and the normalized moments of inertia of said closed contour shape, the bounding box being defined as the ratio of the length to the width of a circumscribed box whose edges are tangent to the contours of said shape;
- in the case where said set of n values is substantially similar to one of said predefined n-tuples, a step for assigning said shape a tag attached to said predefined n-tuple, the set being said to be substantially similar when each of the n values differs only by several percent relative to the values of one of said predefined n-tuples; and
- a step for assigning each of the shapes remaining without a tag at the end of said step for assigning said shape a tag attached to said predefined n-tuple, a tag attached to a predefined normalized invariant vector selected from among a plurality of predefined normalized invariant vectors, minimizing the distance with a normalized invariant vector formed by a predefined number of normalized coefficients representative of the coordinates of said contour in the Fourier space;
(d) a step for identifying said contours of said bone portion including:
- a step for grouping together said shapes so as to form at least one group of shapes delimiting a shared volume isolated in space;
- a step for determining, among said shape groups, a target group of shapes for which the tag corresponding to said bone is primarily associated with the shapes of the group;
- a step for selecting the shapes of said target group of shapes.

2. The identification method according to claim 1, **characterized in that** it further includes a step for replacing the tags of the selected shapes of said target group not corresponding to said predefined bone with said tag corresponding to said predefined bone.

3. The identification method according to any one of claims 1 and 2, **characterized in that** said tags attached to predefined normalized invariant vectors belong to the group including at least:
- long bone;
- short bone;
- elongate bone;
- radiating bone;
- arched bone;
- brittle bone;
- pneumatic bone;
- sesamoid bone.

4. The identification method according to claim 3, **characterized in that** said predetermined number of coefficients is greater than or equal to 8.

5. The identification method according to any one of claims 1 to 4, wherein said predefined bone is a scapula or a humerus.

6. A method for the automatic identification of the surface of at least one singular zone belonging to the group including:
- a protrusion;
- a cavity,
- a substantially flat zone,
over a portion of a predefined bone, the contours of which have been obtained according to any one of the methods according to one of claims 1 to 5, **characterized in that** it includes:
- a step for obtaining the three-dimensional position of at least one specific anatomical point specific to said surface of said portion of a predefined bone from said contours;
- a step for identifying said surface of said singular zone in a spherical cap centered around said point, with a predefined radius and thickness and as a function of the distance of said point from said singular zone and an estimate of the size of said singular zone.

7. The identification method according to claim 6, **characterized in that** said predefined bone being a scapula, said singular zone belongs to the group including:
- acromion;
- glenoid surface;
- coronoid process.

8. A method for automatically determining initial parameters of a passage matrix between a first coordinate system associated with at least one portion of a bone of a patient and a second coordinate system associated with the contours of said bone portion identified in a plurality of images obtained by a medical imaging technique showing sections of a measuring volume including said bone portion, **characterized in that** it includes:
- a step for automatically identifying the surface of a singular zone in said images including the steps of the method according to claim 6;
- a step for determining the barycenter of said singular zone and the vector normal to the median plane of said surface of said singular zone oriented toward the outside, said median plane being an approximation of the direction of the least-squares of the surface of the singular zone;
- a step for obtaining recalibration parameters resulting from the coincidence of said barycenter and said normal vector determined with the barycenter of said singular zone digitized using a laser, using an graphic probe or by feeling on said bone portion and a vector normal to the median plane of the surface of said digitized singular zone, oriented toward the outside;
- a step for forming said initial parameters from said recalibration parameters.

9. A method for simulating the placement of an element of a prosthesis at a singular zone of a portion of a predefined bone of the shoulder including:
- a step for automatically identifying the surface of said singular zone in images of said portion of a predefined bone of the shoulder obtained using a medical imaging technique including the steps of the method according to claim 6;
- a step for determining a first piece of information including the reference plane of said surface, and/or the version angle and/or the incline angle associated with said surface;
- a step for determining a second piece of information representative of the bone capital associated with said surface, from data of said surface and data of the contours of said bone portion obtained according to the identification method according to one of claims 1 to 5;
- a step for retrieving said first and second pieces of information across from at least one geometric parameter of the prosthesis element.

10. A computer program product downloadable from a communication network and/or recorded on a medium readable by computer and/or executable by a processor, **characterized in that** it includes program code instructions for carrying out steps of any one of the methods for the automatic identification of the contours of at least one portion of a predefined bone according to one of claims 1 to 5 and/or steps of any one of the methods for the automatic identification of at least one singular zone of at least one portion of a predefined bone according to one of claims 6 or 7 and/or steps of the method for determining initial parameters of a passage matrix according to claim 8 and/or steps of the simulation method according to claim 9, when said program is executed by a computer.
